## Europäisches Patentamt
## European Patent Office
## Office européen des brevets

(19)

(11) Veröffentlichungsnummer: **0 546 399 B1**

(12)

# EUROPÄISCHE PATENTSCHRIFT

(45) Veröffentlichungstag der Patentschrift: **20.09.95**

(21) Anmeldenummer: **92120305.5**

(22) Anmeldetag: **27.11.92**

(51) Int. Cl.⁶: **C07C 271/28**, C08G 18/80, C08G 18/10, C08G 18/48

(54) **Ether- und Urethangruppen aufweisende Polyisocyanate, ein Verfahren zu ihrer Herstellung und ihre Verwendung.**

(30) Priorität: **10.12.91 DE 4140660**

(43) Veröffentlichungstag der Anmeldung:
**16.06.93 Patentblatt 93/24**

(45) Bekanntmachung des Hinweises auf die
Patenterteilung:
**20.09.95 Patentblatt 95/38**

(84) Benannte Vertragsstaaten:
**AT BE CH DE ES FR GB GR IT LI NL PT SE**

(56) Entgegenhaltungen:
**EP-A- 0 316 738**
**DE-A- 1 090 196**
**US-A- 3 183 112**
**US-A- 4 385 171**

(73) Patentinhaber: **BAYER AG**

**D-51368 Leverkusen (DE)**

(72) Erfinder: **Schmalstieg, Lutz, Dr.**
**Schnurgasse 45**
**W-5000 Köln 1 (DE)**
Erfinder: **Gruber, Hermann, Dr.**
**Paul-Klee-Strasse 87**
**W-5090 Leverkusen 1 (DE)**
Erfinder: **Riberi, Bernd, Dr.**
**Im Alten Driesch 1**
**W-5068 Odenrthal-Osenau (DE)**
Erfinder: **Nachtkamp, Klaus, Dr.**
**Silcherstrasse 13**
**W-4000 Düsseldorf 13 (DE)**

EP 0 546 399 B1

Rank Xerox (UK) Business Services
(3.10/3.09/3.3.3)

**Beschreibung**

Die Erfindung betrifft Ether- und Urethangruppen aufweisende Polyisocyanate auf Basis Toluylendiisocyanat, ein Verfahren zu ihrer Herstellung und ihre Verwendung als Polyisocyanatkomponente in Ein- und Zweikomponenten-Polyurethanlacken.

Urethangruppen aufweisende Polyisocyanate aus niedermolekularen Polyalkoholen und Toluylendiisocyanat sind seit langem bekannt und werden z.B. in den deutschen Patentschriften 870 400, 953 012, 1 090 196 beschrieben. Derartige Produkte sind von großer Bedeutung im Bereich der Polyurethanlacke und -beschichtungen sowie auf dem Klebstoffsektor. Handelsübliche Produkte werden heutzutage so hergestellt, daß Polyalkohole mit der 5- bis 10-fachen Menge Toluylendiisocyanat umgesetzt werden, worauf sich eine destillative Entfernung des überschüssigen Ausgangsdiisocyanats, vorzugsweise im Dünnschichtverdampfer anschließt. Derartige Verfahren sind beispielsweise in DE-PS 1 090 186 bzw. in US-PS 3 183 112 beschrieben. Die auf diese Weise hergestellten Polyisocyanate des Standes der Technik weisen jedoch zwei grundlegende Nachteile auf:

Die Produkte zeigen in Kombination mit handelsüblichen Polyolen eine relativ kurze Verarbeitungszeit, so daß sie nicht auf allen Anwendungsgebieten optimal einsetzbar sind. Außerdem weisen die Produkte des Standes der Technik eine sehr hohe Schmelzviskosität auf, was zur Folge hat, daß sich Toluylendiisocyanat nur bis zu einem Restgehalt von ca. 0,2 Gew.-% destillativ entfernen läßt. Aus arbeitsplatzhygienischen Gründen werden jedoch heute im allgemeinen Monomerengehalte von weniger als 0,1 Gew.-% angestrebt.

Zum Zwecke der Entfernung von Restmonomeren sind in der Patentliteratur eine Vielzahl von chemischen Nachbehandlungsmethoden beschrieben (z.B. US-PS 3 384 624, DE-OS 2 414 413 oder 2 414 391). Derartige Methoden der Monomerenreduzierung beinhalten jedoch immer zusätzliche und somit teuere Verfahrensschritte, wobei die Eigenschaften der Polyisocyanate in der Regel deutlich verändert werden. So haben sich chemische Nachbehandlungsmethoden bei der industriellen Fertigung von Polyisocyanaten nicht durchsetzen können.

Ether- und Urethangruppen aufweisende Polyisocyanate auf Basis von Polyhoydroxypolyethern un Toluendiisocyanat werden in den Schriften EP-A-316 738 und US-A-4 385 171 beschrieben.

Aufgabe der vorliegenden Erfindung war daher die Bereitstellung von Urethangruppen aufweisenden Polyisocyanaten auf Basis Toluylendiisocyanat, die nicht mit den Nachteilen der Polyisocyanate des Standes der Technik behaftet sind.

Diese Aufgaben konnten mit der Bereitstellung der nachstehend näher beschriebenen Ether- und Urethangruppen aufweisenden Polyisocyanate gelöst werden.

Der Erfindung liegt die überraschende Beobachtung zugrunde, daß Addukte aus Toluylendiisocyanat und bestimmten Polyetheralkoholen, insbesondere Polyethertriolen eine verminderte Reaktivität gegenüber Polyhydroxylverbindungen zeigen und gleichzeitig eine niedrige Schmelzviskosität aufweisen, so daß problemlos eine destillative Monomerenentfernung bis auf einen Restgehalt von weniger als 0,1 Gew.-% möglich ist.

Gegenstand der Erfindung sind daher Ether- und Urethangruppen aufweisende Polyisocyanate auf Basis von Polyhydroxylpolyethern und Toluylendiisocyanat, dadurch gekennzeichnet, daß sie

a) einen NCO-Gehalt von 11,8 bis 14,4 Gew.-%
b) eine mittlere NCO-Funktionalität von 3,1 bis 4,0 und
c) einen Gehalt an freiem Toluylendiisocyanat von weniger als 0,1 Gew.-% aufweisen.

Gegenstand der Erfindung ist auch ein Verfahren zur Herstellung derartiger Polyisocyanate durch Umsetzung von organischen Polyhydroxylverbindungen mit überschüssigen Mengen an Toluylendiisocyanat und anschließender destillativer Entfernung von nicht umgesetztem Überschuß dieses Ausgangsdiisocyanats bis auf einen Restgehalt von weniger als 0,1 Gew.-%, dadurch gekennzeichnet, daß man als Polyhydroxylverbindungen Polyhydroxypolyether des Molekulargewichtsbereichs 350 bis 500 verwendet.

Gegenstand der Erfindung ist auch die Verwendung der neuen Polyisocyanate als Polyisocyanatkomponente in Polyurethanlacken, insbesondere in Zweikomponenten-Polyurethanlacken.

Ausgangsmaterialien für das erfindungsgemäße Verfahren sind Toluylendiisocyanat und Polyetherpolyole.

Als Toluylendiisocyanat kommen insbesondere 2,4-Toluylendiisocyanat und seine technischen Gemische mit bis zu 35 Gew.-%, bezogen auf Gemisch, an 2,6-Toluylendiisocyanat in Betracht.

Die beim erfindungsgemäßen Verfahren einzusetzenden Polyetherpolyole weisen ein aus Hydroxylgruppengehalt und Hydroxylfunktionalität berechenbares Molekulargewicht von 350 bis 500, vorzugsweise 400 bis 470 auf. Bevorzugt werden die entsprechenden Polyethertriole eingesetzt. Diese Polyetherpolyole können in an sich bekannter Weise durch Alkoxylierung geeigneter Startermoleküle oder geeigneter Gemische von Startermolekülen erhalten werden, wobei bei der Alkoxylierung insbesondere Propylenoxid

2

und/oder Ethylenoxid, gegebenenfalls im Gemisch und/oder nacheinander in beliebiger Reihenfolge zum Einsatz gelangen. Besonders bevorzugt werden die entsprechenden durch Propoxylierung von Ethergruppen-freien dreiwertigen Alkoholen erhaltenen Polypropylenoxidpolyether eingesetzt. Bevorzugte Startermoleküle sind Glycerin und/oder Trimethylolpropan.

Zur Durchführung des erfindungsgemäßen Verfahrens werden die Polyetherpolyole mit Toluylendiisocyanat bei Temperaturen von 40 bis 140°C, vorzugsweise 50 bis 110°C umgesetzt. Die Mengen der Reaktionspartner entsprechen dabei im allgemeinen einem NCO/OH-Äquivalentverhältnis von 4:1 bis 20:1, vorzugsweise 5:1 bis 10:1.

Nach der Prepolymerisierung wird der Überschuß an nicht umgesetztem Toluylendiisocyanat durch Vakuum-Dünnschichtdestillation bei 100 bis 180°C, vorzugsweise 120 bis 160°C entfernt. Dabei erhält man die erfindungsgemäßen Polyisocyanate in Form halbharter Harze.

Die erfindungsgemäßen Polyisocyanate weisen im Vergleich zu den Polyisocyanaten des Standes der Technik eine sehr niedrige Schmelzviskosität auf. Die Viskosität bei 100°C beträgt in der Regel weniger als 20 000 mPa.s, vorzugsweise weniger als 10 000 mPa.s. Aufgrund der niedrigen Schmelzviskosität läßt sich monomeres Diisocyanat besonders effektiv durch Vakuum-Dünnschichtdestillation entfernen, so daß Restgehalte an freiem Toluylendiisocyanat von weniger als 0,1 Gew.-% mit den in der Technik üblichen Dünnschichtverdampfern problemlos erzielt werden können.

Die erfindungsgemäßen Polyisocyanate stellen Polyisocyanatgemische dar, deren gelchromatographische Analyse das gleichzeitige Vorliegen von Polyisocyanaten einer Funktionalität von 3 als Hauptkomponente und von Polyisocyanaten höherer Funktionalität als Nebenkomponente aufzeigt. Die aus Isocyanatgehalt und dampfdruckosmometrisch bestimmtem Molekulargewicht errechenbare mittlere Funktionalität liegt daher stets im Bereich von 3,1 bis 4,0, vorzugsweise im Bereich 3,3 bis 3,8.

Zur Anwendung gelangen die erfindungsgemäßen Polyisocyanate im allgemeinen in Form einer Lösung in einem organischen Lösemittel. Geeignete Lösemittel sind z.B. die bekannten Lacklösemittel wie Ethylacetat, Butylacetat, Methoxypropylacetat, Toluol, die isomeren Xylole sowie technische Losemittel wie ®Solvent Naphtha oder ®Solvesso oder Gemische derartiger Lösungsmittel. Überraschenderweise hat sich gezeigt, daß Lösungen der erfindungsgemäßen Polyisocyanate eine im Vergleich zum Stand der Technik deutlich bessere Verdünnbarkeit mit geruchsarmen aliphatischen Lösungsmitteln aufweisen. Solche Lösungsmittel sind beispielsweise Petrolether, Waschbenzin, Leichtbenzin, Lackbenzin sowie verschiedene ®Nappar- und/oder ®Isopar-Typen. Diese Tatsache ist insbesondere für die Anwendung in geschlossenen Räumen von Vorteil.

Die erfindungsgemäßen Polyisocyanate stellen wertvolle Rohstoffe für Ein- und Zweikomponenten-Polyurethan-Lacke dar. Das besonders bevorzugte Einsatzgebiet für die neuen Polyisocyanate ist ihre Verwendung als Polyisocyanatkomponente in Zweikomponenten-Polyurethanlacken.

Die für diese bevorzugte Verwendung bevorzugten Reaktionspartner für die erfindungsgemäßen Polyisocyanate sind die in der Polyurethanlacktechnik an sich bekannten Polyhydroypolyester und -ether, Polyhydroxypolyacrylate und gegebenenfalls niedermolekulare mehrwertige Alkohole. Auch Polyamine, insbesondere in blockierter Form als Polyketimine oder Oxazolidine sind denkbare Reaktionspartner für die erfindungsgemäßen Produkte. Die Mengenverhältnisse, in welchen die erfindungsgemäßen Polyisocyanate und die genannten Reaktionspartner bei der Herstellung von Zweikomponenten-Polyurethanlacken eingesetzt werden, werden im allgemeinen so gewählt, daß auf eine Isocyanatgruppe 0,8 bis 3, vorzugsweise 0,9 bis 1,1 gegenüber Isocyanatgruppen reaktionsfähige Gruppen entfallen.

Bei der Formulierung von Zweikomponenten-Polyurethan-Beschichtungsmitteln aus den erfindungsgemäßen Polyisocyanaten und Polyhydroxylverbindungen wurde überraschenderweise gefunden, daß diese Systeme eine im Vergleich zum Stand der Technik wesentlich verlängerte Topfzeit aufweisen.

Falls eine besonders schnelle Durchhärtung gewünscht wird, können die in der Isocyanatchemie üblichen Katalysatoren mitverwendet werden, wie z.B. Amine wie Triethylamin, Pyridin, Methylpyridin, Benzyldimethylamin, N,N'-Dimethylpiperazin oder Metallsalze wie Eisen(III)chlorid, Zinkchlorid, Zink-2-ethylcaproat, Zinn(II)-2-ethylcaproat, Dibutylzinn(IV)dilaurat oder Molybdänglykolat.

Einkomponenten-Polyurethanlacke, die als Bindemittel und insbesondere Zweikomponenten-Polyurethanlacke, die als Vernetzer erfindungsgemäße Polyisocyanate enthalten, führen zu Lackfilmen einer hohen Abriebfestigkeit bei ausgezeichneter Haftung auf den unterschiedlichsten Substraten. Darüber hinaus zeichnen sich die Lackfilme durch hohe Härte und Elastizität aus.

Die Polyurethanlacke, die erfindungsgemäße Polyisocyanate als Bindemittel bzw. als Härter enthalten, können selbstverständlich die aus der Lacktechnologie üblichen Hilfs- und Zusatzmittel wie Pigmente, Verlaufhilfsmittel, Füllstoffe u.dgl. enthalten.

In den nachfolgenden Beispielen beziehen sich alle Prozentangaben auf das Gewicht.

Beispiel 1
Herstellung eines Polyisocyanates

Zu 3654 g eines Gemisches aus 80 % 2,4-Toluylendiisocyanat und 20 % 2,6-Toluylendiisocyanat werden bei 60°C 440 g eines durch Propoxylierung von Trimethylolpropan hergestellten Polyethers des mittleren Molekulargewichts 440 langsam zugetropft. Nach beendetem Zutropfen wird 2 Stunden bei 60°C nachgerührt. Anschließend wird as Produkt durch Vakuum-Dünnschichtdestillation bei 140°C/13,3 Pa (0,1mm) vom monomeren Diisocyanat befreit. Das erhaltene fast farblose Harz weist eine Viskosität von 4700 mPa.s/100°C auf. Das Produkt wird 80 %ig in Methoxypropylacetat gelöst. Die Lösung besitzt folgende Kenndaten:

| | |
|---|---|
| Festkörpergehalt: | 80 % |
| Viskosität: | 10 500 mPa.s/22°C |
| NCO-Gehalt: | 10,5 % |
| Molekulargewicht: | 1150 g/mol (dampfdruckosmometrisch bestimmt) |
| ber. NCO-Funktionalität: | 3,59 |
| Monomerengehalt: | 0,03 Gew.-% |

Die Lösung läßt sich bis zu einem Gewichtsanteil von 45 % mit Lackbenzin verdünnen`

Beispiel 2
Herstellung eines Polyisocyanates

Beispiel 1 wird wiederholt, jedoch unter Verwendung einer gleichen Menge eines Gemischs aus 65 % 2,4-Toluylendiisocyanat und 35 % 2,6-Toluylendiisocyanat. Das resultierende, lösungsmittelfreie, blaßgelbe Harz weist eine Schmelzviskosität von 5500 mPa.s/100°C auf und wird 80 %ig in Ethylacetat gelöst. Die Lösung besitzt folgende Kenndaten:

| | |
|---|---|
| Festkörpergehalt: | 80 % |
| Viskosität: | 1500 mPa.s/22°C |
| NCO-Gehalt: | 10,3 % |
| Molekulargewicht: | 1160 g/mol (dampfdruckosmometrisch bestimmt) |
| ber. NCO-Funktionalität: | 3,57 |
| Monomerengehalt: | 0,03 Gew.-% |

Die Lösung läßt sich bis zu einem Gewichtsanteil von 50 % mit Lackbenzin verdünnen.

Beispiel 3
Herstellung eines Polyisocyanates

Zu 4698 g 2,4-Toluylendiisocyanat werden bei 60°C 360 g eines durch Propoxylierung von Trimethylolpropan hergestellten Polyethers des Molekulargewichts 360 langsam zugetropft. Nach 2-stündigem Nachrühren bei 60°C wird wie in Beispiel 1 aufgearbeitet. Das erhaltene fast farblose Harz weist eine Schmelzviskosität von 18 400 mPa.s/100°C auf. Das Produkt wird 80 %ig in Ethylacetat gelöst. Die Lösung besitzt folgende Kenndaten:

| | |
|---|---|
| Festkörpergehalt: | 80 % |
| Viskosität: | 3700 mPa.s/22°C |
| NCO-Gehalt: | 11,2 % |
| Molekulargewicht: | 978 g/mol (dampfdruckosmometrisch bestimmt) |
| ber. NCO-Funktionalität: | 3,26 |
| Monomerengehalt: | 0,04 Gew.-% |

Beispiel 4
Herstellung eines Polyisocyanates (Vergleichsbeispiel)

Zu 3864 g einer Mischung aus 65 % 2,4-Toluylendiisocyanat und 35 % 2,6-Toluylendiisocyanat wird bei 80°C eine Mischung aus 252 g Trimethylolpropan und 120 g Diethylenglykol langsam zugetropft. Nach 2-stündigem Nachrühren bei 80°C wird wie in Beispiel 1 aufgearbeitet. Das erhaltene blaßgelbe Harz weist eine Viskosität von über 400 000 mPa.s/100°C auf. Eine 75 %ige Lösung in Ethylacetat besitzt folgende Kenndaten:

| | |
|---|---|
| Festkörpergehalt: | 75 % |
| Viskosität: | 1400 mPa.s/22°C |
| NCO-Gehalt: | 13 % |
| Molekulargewicht: | 787 g/mol (dampfdruckosmometrisch bestimmt) |
| ber. NCO-Funktionalität: | 3,25 |
| Monomerengehalt: | 0,23 Gew.-% |

Die Lösung läßt sich bis zu einem Gewichtsanteil von 15 % mit Lackbenzin verdünnen.

Beispiel 5 (Verwendung)

Die Polyisocyanate aus Beispiel 2 und Beispiel 4 werden jeweils mit einem handelsüblichen Polyesterpolyol (®Desmophen 1300, Handelsprodukt der Bayer AG, OH-Gehalt 4 %) zu einer Gesamtkonzentration von 50 % in Ethylacetat im NCO/OH-Verhältnis 1:1 abgemischt.

| | Polyisocyanat Beispiel 2 | Polyisocyanat Beispiel 4 |
|---|---|---|
| Standzeit (bis zur Gelierung) | 5 Tage | 51 Stunden |
| Trocknung (180 µm) | 13 Stunden | 5,5 Stunden |
| Auslaufzeit (DIN 4) | | |
| sofort | 14 Sekunden | 15 Sekunden |
| 4 h | 14 Sekunden | 16 Sekunden |
| 8 h | 15 Sekunden | 17 Sekunden |
| 24 h | 19 Sekunden | 29 Sekunden |
| 32 h | 20 Sekunden | 48 Sekunden |
| Pendelhärte (nach 7 Tagen RT) | 159 Sekunden | 174 Sekunden |
| Anlösbarkeit (nach 7 Tagen RT) (Toluol, Methoxypropyl-acetat, Ethylacetat, Aceton) | 0/0/0/4 | 0/0/0/3 |

**Patentansprüche**

1.  Ether- und Urethangruppen aufweisende Polyisocyanate auf Basis von Polyhydroxypolyethern und Toluylendiisocyanat, dadurch gekennzeichnet, daß sie
    a) einen NCO-Gehalt von 11,8 bis 14,4 Gew.-%,
    b) eine mittlere NCO-Funktionalität von 3,1 bis 4,0 und
    c) einen Gehalt an freiem Toluylendiisocyanat von weniger als 0,1 Gew.-% aufweisen.

2.  Verfahren zur Herstellung von Ether- und Urethangruppen aufweisenden Polyisocyanaten gemäß Anspruch 1 durch Umsetzung von organischen Polyhydroxylverbindungen mit überschüssigen Mengen an Toluylendiisocyanat und anschließender destillativer Entfernung von nicht umgesetztem Überschuß dieses Ausgangsdiisocyanats bis auf einen Restgehalt von weniger als 0,1 Gew.-%, dadurch gekennzeichnet, daß man als Polyhydroxylverbindungen Polyhydroxypolyether des Molekulargewichtsbereichs 350 bis 500 verwendet.

3.  Verfahren gemäß Anspruch 2, dadurch gekennzeichnet, daß man als Polyhydroxylverbindungen Polyethertriole des Molekulargewichtsbereichs 350 bis 500 verwendet.

4.  Verfahren gemäß Anspruch 2 und 3, dadurch gekennzeichnet, daß man als Polyhydroxylverbindungen Polyethertriole des Molekulargewichtsbereichs 400 bis 470 verwendet, die durch Propoxylierung von Ethergruppen-freien dreiwertigen Alkoholen hergestellt worden sind.

5.  Verfahren gemäß Anspruch 2 bis 4, dadurch gekennzeichnet, daß man als Toluylendiisocyanat 2,4-Toluylendiisocyanat oder dessen technische Gemische mit bis zu 35 Gew.-%, bezogen auf Gemisch, an 2,6-Diisocyanatotoluol verwendet.

6.  Verwendung der Ether- und Urethangruppen aufweisenden Polyisocyanate gemäß Anspruch 1 als Polyisocyanatkomponente in Polyurethanlacken.

7.  Verwendung gemäß Anspruch 6 als Vernetzer in Zweikomponenten-Polyurethanlacken.

**Claims**

1.  Polyisocyanates based on polyhydroxy polyethers and tolylene diisocyanate containing ether and urethane groups, characterised in that they
    a) have an NCO content of 11.8 to 14.4 wt.%,
    b) have an average NCO functionality of 3.1 to 4.0 and
    c) have a free tolylene diisocyanate content of less than 0.1 wt.%.

2.  Process for the production of polyisocyanates containing ether and urethane groups according to claim 1 by reacting organic polyhydroxyl compounds with excess quantities of tolylene diisocyanate and subsequently removing any unreacted excess of this starting diisocyanate by distillation down to a residual content of less than 0.1 wt.%, characterised in that the polyhydroxyl compounds used are polyhydroxy polyethers in the molecular weight range from 350 to 500.

3.  Process according to claim 2, characterised in that the polyhydroxyl compounds used are polyether triols in the molecular weight range from 350 to 500.

4.  Process according to claims 2 and 3, characterised in that the polyhydroxyl compounds used are polyether triols in the molecular weight range from 400 to 470, which were produced by propoxylation of trihydric alcohols containing no ether groups.

5.  Process according to claims 2 to 4, characterised in that the tolylene diisocyanate used is 2,4-tolylene diisocyanate or commercial mixtures thereof with up to 35 wt.%, relative to the mixture, of 2,6-diisocyanato-toluene.

6.  Use of the polyisocyanates containing ether and urethane groups according to claim 1 as a polyisocyanate component in polyurethane lacquers.

7. Use according to claim 6 as a crosslinking agent in two-component polyurethane lacquers.

**Revendications**

1. Polyisocyanates présentant des groupes éther et uréthanne à base de polyhydroxypolyéthers et de toluylènediisocyanates, caractérisés en ce qu'ils présentent
   a) une teneur NCO de 11,8 à 14,4% en poids,
   b) une fonctionnalité NCO moyenne de 3,1 à 4,0 et
   c) une teneur en toluylènediisocyanate libre inférieure à 0,1% en poids.

2. Procédé pour la préparation de polyisocyanates présentant des groupes éther et uréthanne selon la revendication 1, par mise en réaction de composés polyhydroxylés organiques avec des quantités en excès de toluylènediisocyanate et élimination ultérieure par distillation de l'excès n'ayant pas réagi de ce diisocyanate de départ jusqu'à ce qu'on obtienne une teneur résiduelle inférieure à 0.1% en poids, caractérisé en ce que, comme composés polyhydroxylés, on utilise des polyhydroxypolyéthers du domaine de poids moléculaire de 350 à 500.

3. Procédé selon la revendication 2, caractérisé en ce que, comme composés polyhydroxylés, on utilise des polyéther-triols du domaine de poids moléculaire de 350 à 500.

4. Procédé selon les revendications 2 et 3, caractérisé en ce que, comme composés polyhydroxylés, on utilise des polyéther-triols du domaine de poids moléculaire de 400 à 470, qui ont été préparés par propoxylation d'alcools trivalents exempts de groupes éther.

5. Procédé selon les revendications 2 à 4, caractérisé en ce que, comme toluylènediisocyanate, on utilise le 2,4-toluylènediisocyanate ou ses mélanges techniques avec jusqu'à 35% en poids, rapportés au mélange, du 2,6-diisocyanatotoluène.

6. Utilisation des polyisocyanates présentant des groupes éther et uréthanne selon la revendication 1, comme composant de polyisocyanate dans des laques, des vernis ou des peintures de polyuréthanne.

7. Utilisation selon la revendication 6, comme agent de réticulation dans des laques, des vernis ou des peintures de polyuréthanne à deux composants.